Europäisches Patentamt

European Patent Office

Office européen des brevets

(19)

(11) Publication number:
**0 327 395**
A2

## (12) EUROPEAN PATENT APPLICATION

(21) Application number: **89301099.1**

(22) Date of filing: **03.02.89**

(51) Int. Cl.⁴: **G 01 N 33/53**
G 01 N 33/543

(30) Priority: **05.02.88 US 152515**

(43) Date of publication of application:
**09.08.89 Bulletin 89/32**

(84) Designated Contracting States:
**AT BE CH DE FR GB IT LI NL SE**

(71) Applicant: **IDEXX CORP.**
**100 Fore Street**
**Portland Maine 04101 (US)**

(72) Inventor: **McMahon, Philip**
**45 Winn Road**
**Falmouth Maine (US)**

**Workman, Erwin**
**6 Roundabout Lane**
**Cape Elizabeth Maine 04107 (US)**

**Brooks, Paul Christopher**
**922 Baxter Boulevard**
**Portland Maine 02183 (US)**

(74) Representative: **Hitchcock, Esmond Antony et al**
**Lloyd Wise, Tregear & Co. Norman House 105-109 Strand**
**London WC2R 0AE (GB)**

(54) **Assay kit and method.**

(57) A kit, or workstation, suitable for assay of a member of a specific binding pair in a sample, the assay entailing performance of a plurality of steps. The kit includes a first unit having at least one dry reagent, suitable for use in one the steps of the assay reaction, bound to a solid surface. Also provided is a second unit including a plurality of wells, wherein one of the wells is adapted to hold liquid suitable for reconstituting the dry reagent, or for use in a one of the assay steps. Also provided is means for causing the first and second units to be brought together so that the dry reagent and the liquid suitable for reconstituting it are brought in close proximity. The dry reagent is reconstituted by the liquid before or during one of the assay steps. When the first and second units are brought together they form a workstation adapted to provide a reagent for each of the steps of the assay. A test device may be positioned over or within each of these wells in a predetermined order to carry out the steps of the assay.

EP 0 327 395 A2

**Description**

## ASSAY KIT AND METHOD

This invention relates to assays for antigens, antibodies or nucleic acids in a sample, e.g. a biological fluid such as whole blood, serum, plasma, and urine.

Generally a wide variety of substances are commonly detected or measured by immunoassay methods in biological samples; examples are hormones, antibodies, toxins, and drugs. Usually, though not always, either substance being detected, or a substance used in the detection, is an antibody, hence the term "immunoassay". The antibody is a member of a specific binding pair, the other member of the pair being referred to as an antigen or analyte. Other specific binding pairs, besides antibody-antigen pairs, which are measured and used in immunoassays, include pairs of molecules which have specific binding affinity for each other, e.g. hormones--hormone receptors, and biotin--avidin. Similar methods are used in detection of nucleic acids, where a specific nucleic acid may be specifically detected by use of an oligonucleotide able to hydrogen bond with the nucleic acid to be detected. Examples include DNA and RNA molecules.

Immuncassays are commonly carried out, at least in part, on solid supports, e.g., glass fiber membranes. The two most common formats for immunoassays employing solid supports are competitive and sandwich formats. In a typical competitive assay, she substance to be measured (the analyte) is a low molecular weight substance such as a drug residue or a hormone, with a molecular weight from about 100 to about 2000; such low molecular weight substances do not easily lend themselves to sandwich assays, described below. In a typical competitive assay, an antibody to the analyte is immobilized on a solid support, and the sample suspected of containing the analyte is brought into contact with that solid support. At the same time, or at a later time, a liquid solution containing labeled analyte is contacted with the support, so that the labeled analyte and any analyte in the sample compete for binding to the immobilized antibody. (If the substance being measured is itself an antibody, the immobilized analyte can either be antibody to that antibody, or an antigen for which that antibody is specific.) The solid support is then washed and the amount of label measured or detected as an inverse measure of analyte in the sample. Typically the label is a chemiluminescent substance, a radioisotope, or most preferably an enzyme which in the final step reacts with a chromogenic substance which develops color of intensity inversely related to the amount of analyte present in the sample. A typical competitive format is described e.g., in Littman et al., U.S. Patent No. 4,540,659, hereby incorporated by reference.

Sandwich assays (e.g., as described in David et al., U.S. Patent No. 4,376,110, hereby incorporated by reference) generally are used to detect or measure substances, (again, analytes) of molecular weights above 2000, e.g., antibodies or other proteins. In a typical sandwich assay, a first antibody to the analyte is immobilized on a solid support, which is then contacted with the sample so that any analyte in the sample binds to the antibody. A second, labeled antibody to the analyte is then added, the support is washed, and the amount of bound label is measured, bound label being proportional to the amount of analyte in the sample.

Nucleic acid assays are commonly carried out, at least in part, on a solid support or in a liquid format. The solid support is commonly nitrocellulose, or a reactive paper, such as DBM paper. The nucleic acid to be detected, or the nucleic acid being used for its detection, i.e., as a probe, may be bound to this solid support, and the other member of these binding nucleic acids will be in solution. The two nucleic acids are permitted to react to form duplex nucleic acid which can then be detected either directly by detecting the duplex nucleic acid, or by detecting a label on one or both of the nucleic acids.

Commonly, the above assays are performed by skilled laboratory staff or technicians using complex apparatus and necessitating measurement of small amounts of liquid reagents. Some manufacturers now provide home assay kits in which the user is provided with a series of pre-made reagents suitable for use in the assay.

There are a number of aspects of the present invention which provide assay kits which are superior to prior assay kits in various ways.

In a first aspect, the invention features a kit, or workstation, suitable for assay of a member of a specific binding pair in a sample, the assay entailing performance of a plurality of steps. The kit includes a first unit having at least one dry reagent, suitable for use in one of the steps of the assay reaction, bound to a solid surface. Also provided is a second unit including a plurality of wells, wherein one of the wells is adapted to hold liquid suitable for reconstituting the dry reagent. The first and second units are adapted to be brought together so that the dry reagent and the liquid suitable for reconstituting it are brought in close proximity such that the dry reagent is reconstituted by the liquid before or during one of the assay steps. When the first and second units are brought together they form a final workstation adapted to provide a reagent for each of the steps of the assay. A test device may be positioned over or within each of these wells in a predetermined order to carry out the steps of the assay.

In preferred embodiments, the first and second units are joined together by a hinge which allows the first unit to be positioned over the second unit; the second unit has separating means positioned over the well to prevent liquid within the well from evaporating; the well has absorbent material suitable for preventing the liquid from spilling from the well; the well has a plastic grid to aid in mixing the dry reagent and the liquid; the first unit includes a well adapted to hold the sample; the dry reagent is held within a liquid permeable solid, most preferably the solid material is felt; the dry reagent is a lyophilized reagent; the second unit includes a well adapted to hold the sample, and the first unit includes a second well adapted to fit within this sample well, and this second well has a prefilter positioned to filter liquid from the sample well into the second well; the first unit and the second unit can be brought together in only one orientation, and in this orientation the dry reagent and the liquid are brought in close proximity to each other; the first unit has snap-locking means, the second unit has mating means, and these are fixedly connected together when the first and second units are brought together; when the test device is brought in contact with the dry reagent in the first unit this contact causes reconstitution of the reagent; the kit is designed for an immunoassay, most preferably the immunoassay is a competitive or sandwich immunoassay; the first unit has two dry reagents, wherein one reagent is a conjugate of a specific antibody and a detectable enzyme, and the second reagent is a substrate for the enzyme; most preferably the enzyme is alkaline phosphatase and the substrate is indoxyl phosphate; the first unit has at least two dry reagents, and the second unit has four wells, including a sample well; most preferably the second unit also has a fifth well which holds a stop solution; the second unit has heating means, most preferably the heating means is a holding means containing two separated chemicals which, when mixed together, react in an exothermic reaction; even more preferably the exothermic chemicals are diatomaceous earth or sodium hydroxide.

In a second aspect, the invention features a method suitable for the assay of a member of a specific binding pair in a sample. The assay entails performance of a plurality of steps. The method includes the steps of a) providing a first unit having at least one dry reagent for use in one assay step, bound to a solid surface, b) providing a second unit having a plurality of wells, wherein one well is adapted to hold liquid for reconstituting the dry reagent, or is of use in one of the assay steps, c) positioning the first unit and the second unit together so that the dry reagent and the well, adapted to hold the liquid for reconstituting the dry reagent, are brought in close proximity, d) providing a test device which may be positioned over or within each well, and e) positioning the test device over the well having the liquid to allow reaction of the test device with the dry reagent and the liquid after the dry reagent and the liquid have been reconstituted.

In preferred embodiments, the method involves positioning the test device in each well in a predetermined order to carry out each step of the assay.

Assays can be performed, according to the invention, by any lay person without the person having knowledge of the mechanism by which the assay works, and without the person having to perform any measuring steps, except in some cases on the sample itself. The kit is in a shelf stable format, thus providing improved stability of the assay materials, and allowing the assay kit to be stored for several months, or even years, prior to its use. The kit can be provided with or without the necessary liquid reagents. Preferably, the liquid reagents are provided to ensure that even the simple steps of adding defined reagents to specific wells are performed by competent personnel prior to running of the assay reaction.

The kit can be used in any desired format for immunoassays, or assays for nucleic acids. For example, positive results can be observed using enzymatic detection tests, or by measuring the conductivity, the chemiluminescence, or the radioactivity of the final reactants. Thus the assay is suitable for any reaction chemistry that is presently known. Further, the kit can be provided with automated means for providing heat, to heat any of the reactants which require it, in a specific and predetermined manner.

One particular kit format that can be used is the one disclosed in commonly owned applications, Brooks et al. USSN 118,750, filed November 6, 1987 and in PCT/US88/04059. That kit generally features a dipstick test device having a control absorbent in liquid-transferring contact with an aqueous permeable, aqueous insoluble reaction zone, adapted to retain the detectable reaction product formed when analyte in the sample is treated with at least one liquid reagent. The control absorbent has a predetermined, limited, standard, liquid-absorbing capacity, and it is adapted to absorb a predetermined volume of liquid from the sample after the sample passes through the reaction zone. That predetermined, limited, standard volume is selected to enable reliable detection of the analyte, taking into account the reagent volumes and concentrations present, the mode of detecting the reaction product, and the analyte concentration level at which discrimination is desired. The control absorbent and the means defining a reaction zone are positioned in the device in the manner that, by filling the control absorbent to capacity, it effectively meters a predetermined limited flow between a first region of the reaction zone, which is exposed to receive liquid sample, and a second region thereof, spaced from the first region, which is in liquid transferring relation with the control absorbent.

A second aspect of the dipstick generally features a device that includes: an absorbent reservoir; means retaining the absorbent reservoir in a first position spaced apart from liquid-transferring contact with the control absorbent or the reaction zone; and means moving the absorbent reservoir from the first position to a second position, in which the reservoir is in liquid-transferring contact with the control absorbent and/or the reaction zone.

The two aspects of the dipstick can be combined in a single device so that the control absorbent draws the predetermined sample volume into the reaction zone while the absorbent reservoir is in the first position; then the absorbent reservoir is moved into the second position, and the means defining a reaction zone is contacted with at least one of the liquid reagents which is drawn through the reaction zone into the absorbent reservoir.

Preferred embodiments of the dipstick include the following features. In the first position the absorbent reservoir is retained above the reaction zone, and it is moved from the first position to the second position, where it rests and is retained in liquid transferring contact above the reaction zone. A plurality of liquid reagents can be drawn upwardly through the reaction zone into the absorbent reservoir without additional reservoir movement. A latch means can be used to restrain movement of the absorbent reservoir from the second position back to the first position, so a plurality of liquid reagents can be introduced to the reaction zone while the absorbent reservoir is latched in the second position. The dipstick device also can include a means to release the latch after the detectable product is formed, so the absorbent reservoir is returned to the first position while the reaction product is being detected. At least one reactant participating in a reaction to form the detectable product is included in the reaction zone; for example, a specific binding partner for the analyte can be included in the reaction zone to trap the analyte there. The reaction zone is impermeable to the analyte. The reaction zone is defined on a flat member, and the two regions of the zone are opposite faces of the member. The reaction product may be detected by a characteristic of the product, such as color intensity, optical density, reflectance density, pH, fluorescence, or conductivity. For example, it may be detected by external visual inspection of the means defining a reaction zone. A contrast region surrounding said reaction zone aids detection by contrasting with the reaction zone in respect to the characteristic detected. An intensity scale may be included to aid quantative detection of sample analyte. The reaction zone is contained in a test head, and the absorbent reservoir is concentrically positioned with respect to the test head, allowing telescoping movement of the absorbent reservoir with respect to the test head. The test head includes at least one control region and at least one reaction zone, with dividers isolating each control region and each reaction zone. Also, the test head comprises an aqueous impermeable face plate having at least one opening to allow liquid to reach the reaction zone, and a second opening to allow liquid to read the control zone. The test device is accompanied by a reagent pack sized and configured to supply a plurality of reagents to the reaction zone. To detect the detectable reaction product, the device includes a developer selected to participate in generating a colored substance from a chromophore.

Preferably, while the absorbent reservoir is retained in the first position, the sample is contacted with the reaction zone, thereby flowing the predetermined limited sample volume through the means defining the reaction zone; thereafter, the absorbent reservoir is moved in contact with the control absorbent; while the absorbent reservoir is maintained in contact with the control absorbent, the reaction zone is contacted with at least one of the liquid reagents, which flows through the reaction zone into the absorbent reservoir. While the absorbent reservoir is maintained in the second position, the reaction zone may be contacted with one or more additional reagents which are allowed to flow through the reaction zone into the absorbent reservoir. A predetermined time is allowed after the sample absorption and before moving the absorbent reservoir into the second position, to allow the analyte to react with reactant in the reaction zone. After the detectable product is formed, the absorbent reservoir may be returned to the first position to enable detection of the reaction product while the absorbent reservoir is in the first position, i.e., in the absence of continued flow through the reaction zone caused by the absorbent reservoir. The absorbent reservoir can be maintained in the first position for a predetermined length of time, selected to control the desired reaction, so that both the volume of sample contacted with the reaction zone and the duration of that contact are controlled. The test head of the device is at one end and is positioned downwardly to be immersed in the sample and the liquid reagent(s). Then, after the detectable product is formed, the device is inverted to position the test head on top during detection of the product.

Other features and advantages of the invention will be apparent from the following description of the preferred embodiments thereof,

The drawings will first briefly be described.

Drawings

Fig. 1 is a perspective view of one embodiment of a workstation of the invention;

Fig. 2 is an exploded perspective view of said workstation;

Fig. 3 is a perspective view showing assembly of the final workstation;

Figs. 4, 5 and 6 are perspective views showing use of the final workstation;

Fig. 7 is a diagrammatic representation of the two parts of the final workstation;

Fig. 8 is a perspective view of an alternative workstation;

Fig. 9 is a perspective view showing use of the alternative workstation; and

Fig. 10 is an exploded view of a dipstick device suitable for use with the workstations of this invention.

Structure -- hCG Kit

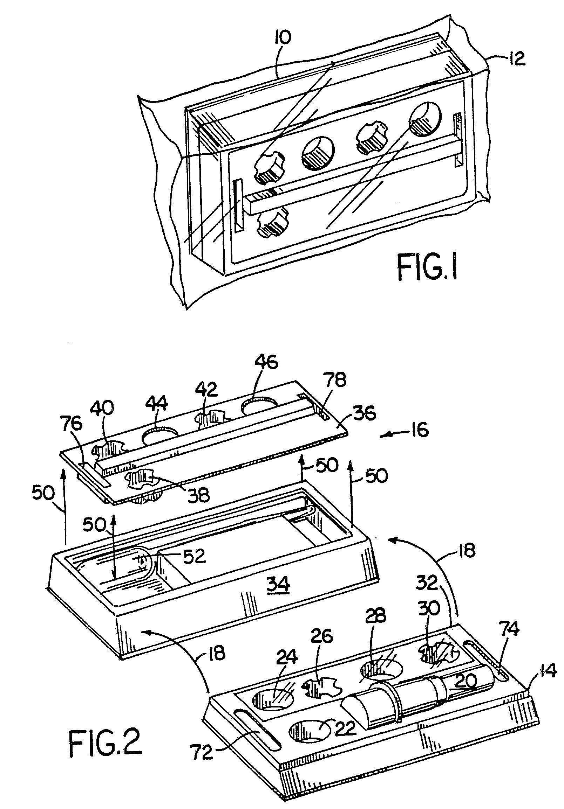

Referring to Fig. 1, workstation 10 is used to perform a sandwich assay for human B-chorionic gonadotrophin (hGG) in urine. Two antibodies are used in the assay, one is a polyclonal antibody to hCG (obtained from Immunosearch, Toms River, NJ), the other a monoclonal antibody to hCG conjugated with alkaline phosphatase (obtained from Immunosearch, NJ). The alkaline phosphatase is detected using the chromogenic substrate indoxyl phosphate.

Workstation 10 is held within sealed plastic container 12. This is the form in. which workstation l0 is stored and provided to a user. The container contains a desiccant pouch to absorb any moisture inside the container. Prior to use, plastic container 12 is severed so that workstation 10 can be removed from it.

Referring to Figure 2, when workstation 10 is removed it can be separated into two components, a lower unit 14 and upper unit 16, as shown by arrows 18. Lower unit 14 includes a dipstick device 20, constructed as described in Brook et al., supra, and a series of five wells 22, 24, 26, 28, and 30. Wells 24, 26, 28, and 30 are covered by a transparent plastic 32 which is tightly sealed to lower unit 14. Upper unit 16 consists of two separable components, a container 34 and a shelf 36. Shelf 36 is provided with three well-like protrusions, termed wells 38, 40, and 42 and two apertures 44 and 46. It also is provided with a handle 48 suitable for removing shelf 36 from container 34 as shown by arrows 50. Shelf 36 also has two elongated projections 76, 78 which are suitably sized to snap-fit into two elongated apertures 72, 74 provided in lower unit 14. Within container 34 is a pipet device 52, suitable for transferring a sample solution to a sample well 38.

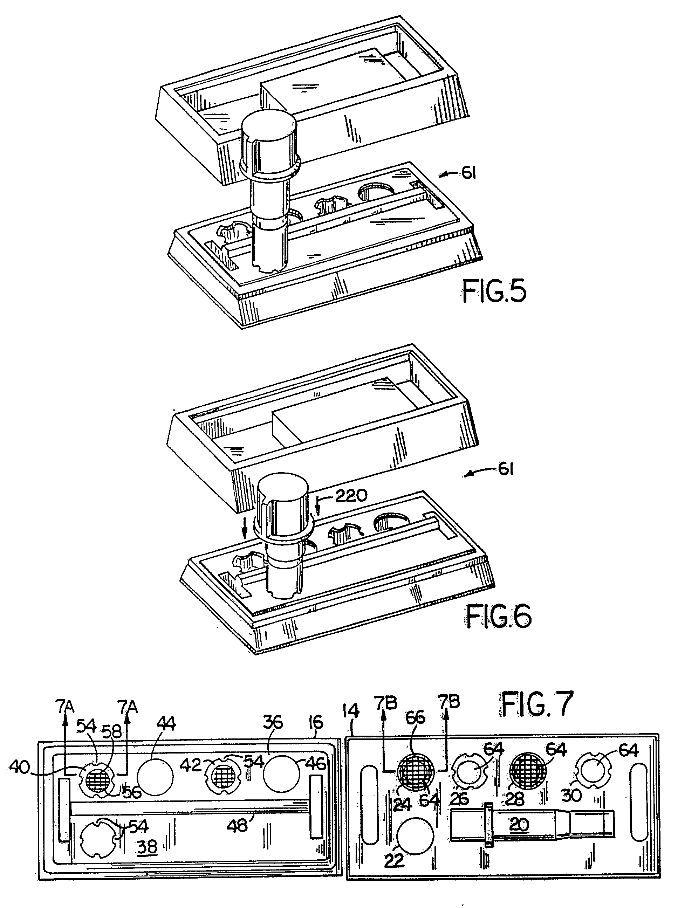

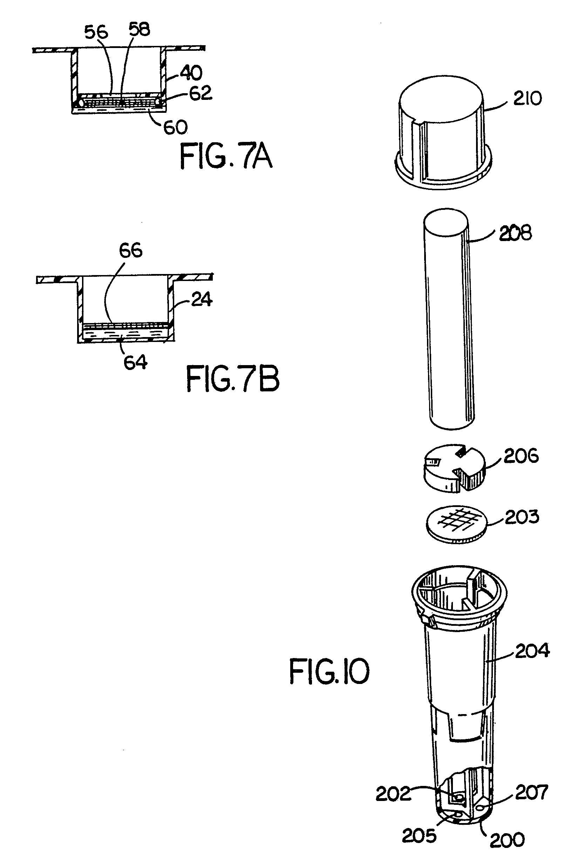

Referring to Fig. 7, a detailed view of unit 14 and shelf 36 is shown. Well 38 is simply an empty plastic well having four protrusions 54 which serve to prevent the distal end of dipstick device 20 from contacting the bottom of the well 38 when the dipstick is inserted within the well. These protrusions are provided in all wells of the workstation. They prevent damage to the filters at its end of dipstick 20 (see below). Also provided in wells 40 and 42 are apertures 56 below which is mounted a plastic grid 58, and a felt disk of one centimeter diameter. These components are shown in detail in Fig. 7A, where felt disk 60 is secured to well 40 by diluted adhesive (Elmers Glue diluted 1:1 with distilled water) 62. Grid 58 is held in place against felt 60 and well 40. Polypropylene felt disk 60 (obtained from Troy Mills, NH) in wells 40 and 42 contains a dried or lyophilized reagent suitable for use in the assay reaction. Well 40 contains dried conjugate which will be reconstituted by liquid within well 24. The dried conjugate is formed as follows 0.45 ml of 40-50µg/ml conjugate is dried in a diluent solution of 0. 01M Tris pH7.4, 50% calf serum, thimerosol (0.1%) and gentamycin (0 002%) onto the 1 cm felt disk. The disk is lyophilized to dryness. Well 42 contains substrate (indoxyl phosphate, 3.1 g/l in solution containing Tris (0.5M), Na azide (0.1%), NaCl (1%), 2-amino, 2 methyl, 1-propanol (1%), pH 10.0.) lyophilized onto felt 60 (using a Virtis lyophilizer)

Referring again to Fig. 7, well 22 is simply a well formed of large enough diameter to allow well 38 of upper unit 16 to be inserted within it. Similarly, wells 24 and 28 are of a diameter (eg., 11/16″) suitable for insertion of wells 40 and 42, respectively. The volume (1.2 ml) of wells 40, 42 is great enough to prevent overflow of liquid within wells 24, 28, when the wells are pushed together. Further, the wells are shaped to mate in a sealing fashion to cause liquid from wells 24, 28 to be forced into wells 40, 42. Wells 24, 26, 28 and 30 each contain a felt disk 64 of about 1 centimeter diameter. Wells 24 and 28 further contain a fiberglass grid 66 (common fiberglass window screen) positioned above polypropylene felt disk 64, as shown in detail in Fig. 7B. This grid aids in mixing and reconstitution of the dried reagent in the felt with reconstituting liquid in the wells. The felt in well 24 acts to prevent liquid moving around the well during storage and transport, and to retain it in the bottom of the well.

Each of wells 24, 26, 28 and 30 is provided with a suitable volume of liquid suitable for use in the assay reaction. The liquid in well 24 is 20-25 50µl drops of distilled water containing 0.1% azide as preservative. Well 26 contains 1 ml of a wash solution suitable for removing excess conjugate. It contains BSA (5%), NaCl (1M), dry milk (2%), thimerosal (0.1%), and gentamycin (0.002%).

Well 28 contains distilled water, identical to that in well 24, suitable for reconstituting the substrate. Finally, well 30 contains 0.75 ml of a stop solution suitable to change the pH of the membrane on dipstick 20 to about pH4.5 or 4.6. This inactivates the alkaline phosphatase. The stop solution is molybdic acid buffer (0.1 M).

Assembly of Final Workstation

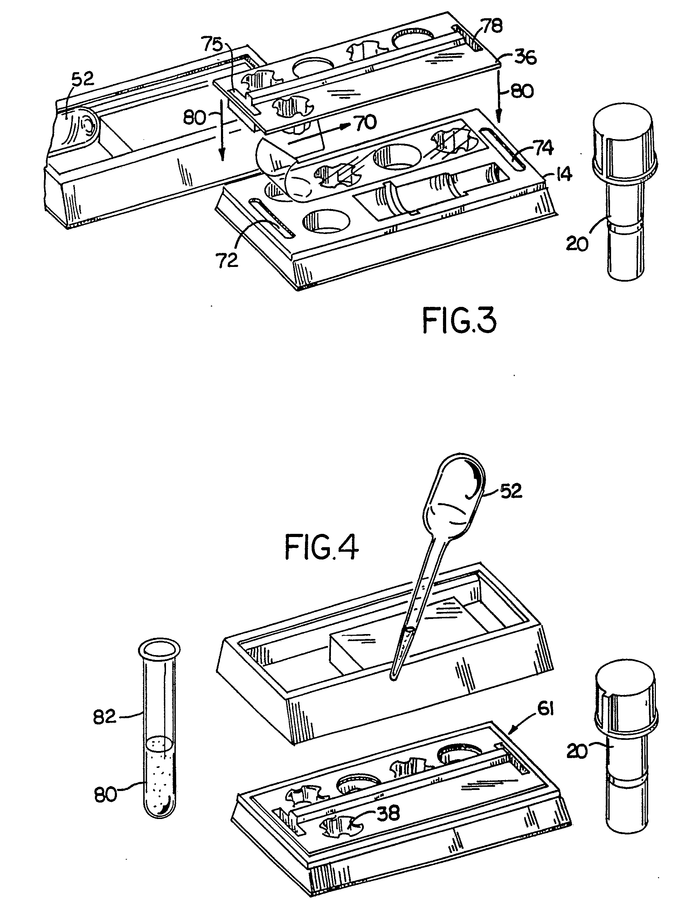

Referring to Fig. 3, after upper unit 16 has been removed from lower unit 14, the user peels transparent plastic 32 from wells 24-30, as shown by arrow 70, to expose the liquid in these wells. Dipstick device 20 is also removed from lower unit 14. At this point shelf 36 can be positioned over lower unit 14 such that wells or apertures 38, 40, 42, 44, and 46 are positioned over wells or apertures 22, 24, 26, 28, and 30, respectively, and such that apertures 72 and 74 in unit 14 are permitted to mate with protrusions 76 and 78, respectively, in unit 36. The apertures and protrusions are pushed together as shown by arrows 80 and snap-fit together. The completed assembly is shown in Fig. 4 where wells 38, 40, and 42 are held within apertures and wells 22, 24, and 28 and wells 26 and 30 are accessible through apertures 44 and 46, respectively. The final workstation 61 is now ready for use to perform an assay.

When shelf 36 is positioned within lower unit 14 the liquid within wells 24 and 28 reacts with dried reagents impregnated in disks 60 and reconstitutes the reagents. Such reconstitution is instantaneous in this embodiment.

The structure and working of dipstick device 20 will now briefly be described. Referring to Fig. 10, this device is shown in exploded form. At the distal tip 200 of the device are three circular apertures 202, 205, 207 over which is positioned a glass fiber membrane 203. Each aperture allows access to parts of this membrane. Reagents suitable as a positive control, a negative control, and a test area for the assay are placed through these apertures. The reagents on these areas are described below. The filter is held within a plastic tube 204 and placed next to an absorbent reservoir 206, which is designed to cause exactly 25µl of liquid to be taken up through each of apertures 202, 205, 207 when dipstick device 20 is placed within a liquid. A second absorbent 208 is positioned at a distance from reservoir 206 when cap 210 is in its resting position. However, when cap 210 is depressed, it pushes absorbent 208 against absorbent reservoir 206 and allows up to 5 ml of liquid to be taken up through apertures 202, 205, 207 when distal end 200 is immersed in a liguidN The rate of uptake is constant at about 500μl per minute.

Polyclonal antibody to hCG is bound to filter 203 at aperture 202 (the test spot). In the assay, described below, this antibody binds hCG in the sample. The bound hCG is then reacted with conjugate and excess conjugate washed off. The more hCG present in the sample, the more conjugate that will bind. The conjugate is then allowed to react with substrate to produce a color reaction. The more color, the more hCG in the sample.

Two control spots are also provided on dipstick device 20 at apertures 205 and 207. The positive control spot 205 is provided with βhCG so that a color reaction will be observed if the conjugate and substrate are functioning properly. The negative control spot 207 is provided only with buffer and nonspecific protein and thus will not produce a color reaction unless the conjugate reads non-specifically.

To prepare the test spot, 200ug/ml of the polyclonal antibody is coated onto 0.8 micron latex particles (Pandex Labs. IL) in 0.01M potassium phosphate buffer, pH7.2, by overnight adsorption, overcoated with the same buffer containing 5% bovine serum albumin for 30 min. to 2 hours, and then washed by centrifugation at 10,000 g for 10 min, and resuspension in the original volume of spotting buffer (0.01M potassium phosphate containing 2.5% sucrose and 0.1% Tween 20). 4μl of a 0.5% solution of these particles is spotted onto device 20 through one of apertures 202, the test spot. To prepare the control spots, 100 mlu of hCG is similarly bound with 0.5% latex particles (30 min. to overnight), washed in spotting buffer by centrifugation at 10,000 rpm for 10 min. and resuspending in the original volume of spotting buffer, to give 0.5% particles and 4 μl spotted through positive control aperture 205, and 4 μl, latex particles having bound bovine serum albumin is spotted through negative control aperture 207, with latex particles prepared as above, except lacking hCG. After spotting, the filter is dried at room temperature for 5 minutes.

LCG Assay Method

Referring again to Fig. 4, in order to commence an assay using final workstation 61 a sample 80, usually of urine, is obtained in any standard manner and placed within a convenient receptacle 82. A sample is then withdrawn using pipette 52, provided with workstation 10, and about 1 ml of sample dispensed into well 38. Dipstick device 20 is placed within this well for 2 minutes to pick up 75μl of urine. The dipstick is removed from the well, its cap depressed, and the dipstick then placed into well 40 to pick up conjugate for 2 minutes. This allows almost all of the liquid (about 0.5 ml) in the well to be taken up. The dipstick is then moved to well 26 to wash the filter for one minute. Again, almost all the liquid is also taken up from this well. Following the wash, dipstick 20 is placed in well 42 to pick up substrate. After 30 seconds, the device can be removed to determine if a visible spot has been developed. If one is visible, then the reaction can be stopped by placing dipstick 20 within well 30 for 15-30 seconds. If no reaction is seen, incubation can be continued for up to 5 minutes to assure detection of even low concentrations (e.g. 5 mIU) of hCG.

Structure -- Brucella Kit

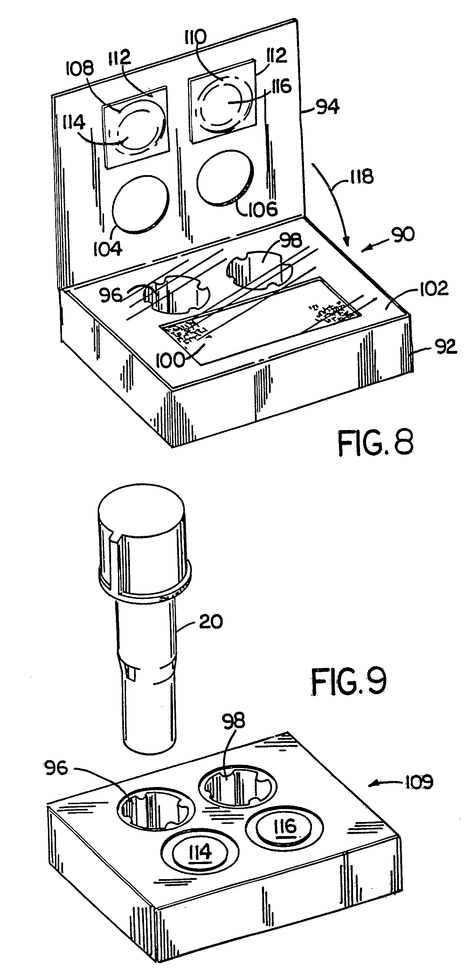

Referring to Figs. 8 and 9, a device 90 suitable for performing a sandwich immunoassay to detect Brucella abortus antigen is shown. Device 90 is provided with a lower container 92 and an upper lid 94. Lower container 92 has two wells 96 and 98 and a pad blotter 100, formed of felt material. Wells 96 and 98 and pad blotter 100 are covered by a transparent mylar plastic 102 which is sealed against lower unit 92. Upper unit 94 has two apertures 104, 106, and two other apertures 108, 110, covered by a non-woven polyester mesh 112, which holds a glass fiber filter 114 or 116 (Gelman Sources, AnnArbor, MI) in position within apertures 108 and 110. Within these glass fibers are held dried reagents suitable for use in an immunoassay. Well 96 is empty, whereas well 98 contains 1 ml wash solution described above for use in the assay. This solution is also suitable as a stop solution. Pad blotter 100 contains about 3 ml water and azide (0.1%) for reconstituting dried reagents in filters 114, 116.

Filter 114 contains a conjugate which is a conjugate consisting of antibody able to bind bovine IgG which is bound by the Brucella antigen, lipopolysaccharide (LPS), conjugated to alkaline phosphatase (obtained from Kirkegaard and Perry Labs. MD). The conjugate is provided at 10μg/ml as a 1 in 75 dilution in the diluent solution described above, three drops of which are lyophilized on the dipstick filter 203. Filter 116 is provided with three 50 μl drops of indoxyl phosphate (described above) as substrate lyophilized onto the filter.

Dipstick 20 again has three spots. The sample spot 102 contains LPS isolated, for example, by phenol extraction of Brucella abortus cells. The LPS is adsorbed onto latex particles of 0.8 microns at a concentration of 1%, and overcoated with 2% fetal bovine serum (FBS) in 0.01 M phosphate buffer, pH 7.2. The particles are then diluted in spotting buffer containing 2% FBS, 2.5% sucrose and 0.05% Tween 20. The positive control spot 105 contains 0.8 micron latex particles coated with bovine IgG at 100 μg/ml (Sigma Chemical Co., St. Louis, MI) overcoated as described above, and diluted in spotting buffer. The negative control spot 107 contains 0.8 micron latex particles coated in FBS and diluted into spotting buffer.

Prior to use, plastic membrane 102 is peeled from lower unit 92 to expose wells 96, 98, and pad blotter 100. Upper lid 94 is moved as shown by arrows 118, to form a closed structure, shown in Fig. 9, which is a final workstation 109. In this position apertures 104, 106 allow access of device 20 to wells 96, 98.

Brucella Assay Method

During use, a sample, usually bovine serum diluted 1:20 in 2% FBS in 0.01 M potassium phosphate buffer, pH 7.2, is placed within well 96. The assay procedure is performed as follows: briefly, dipstick 20 is inserted into well 96 and allowed to pick up 75µl of the sample. Dipstick 20 is then removed, its cap depressed, and the distal portion of dipstick 20 pushed against filter 114 for 2 minutes to allow reaction of antibody bound to the <u>Brucella abortus</u> LPS to react with the conjugate. The conjugate is reconstituted by water from pad 100 when dipstick 20 pushes filter 114 against it. After the reaction, dipstick 20 is inserted into well 98, which contains wash solution, for a period of 30 seconds. Finally, dipstick 20 is pushed against filter 116 to allow the lyophilized substrate to be reconstituted and then react with the bound conjugate. After 30 seconds to 3 minutes the results of the assay can be read from dipstick 20. The reaction may be stopped by placing dipstick 20 within the wash solution for a second time, thus changing the pH of the filter and inhibiting the alkaline phosphatase reaction.

### Other Embodiments

Other embodiments are within the following claims. For example, an improvement on the design of workstation 10 is to form sample well 38 with an aperture, as in wells 40 and 42, and to fix a prefilter to this aperture. Further, aperture 22 can be replaced with a well, and the sample placed within this well. When shelf 36 and cover unit 14 are connected, the sample in the new well will be filtered through the prefilter into well 38. This format is suitable for assays on diluted blood, where red blood cells must be removed.

The above devices are both designed so that the dry reagents can only be positioned over the reconstituting liquids in one way. This ensures that the user, who may have no knowledge of the mechanism of the assay, causes reconstitution to occur appropriately. Other mechanisms for achieving this are well known in the art, including hinge devices, and protrusions to prevent improper mating of the component parts of the device. Further, labels may be permanently fixed to each well to ensure that the order of use of the wells is correctly followed by the user.

Another format includes provision of a filter 114 or 116 held removably on one part of the device. Thus, pressure from dipstick 20 against this filter will cause it to be dislodged into a well below, and allow reconstitution by the liquid in the well. Such a format ensures that reconstitution occurs only just before the reagent is needed it also ensures that a known amount of the reagent will be taken up by the dipstick device, since the reagent will be reconstituted at a predetermined concentration. Thus, this format has the advantages of both the above embodiments.

The devices of this invention can be used in any number of immunoassays or other assays for members of a specific binding pair, e.g., DNA and RNA molecules or penicillin-binding-proteins and penicillin. By simply altering the antibodies, antigens and detection reagents (e.g., enzymes) the workstation can be adapted for any specific assay. Further, the assay may be used for competitive as well as sandwich assays. For example, in a competitive assay, there are two types of formats. The first, an uptake format, encompasses firstly contacting the bound member of the binding pair of the dipstick spot 202 with the sample so that the unbound member, which is to be detected, can bind all available sites. If this unbound member is at a low concentration then not all available sites will be bound. The dipstick is then reacted with a conjugate of unbound member and a detectable reagent (e.g., an enzyme), which binds to any unbound sites. The amount of conjugate bound can then be measured by reaction with substrate. In this assay, the format used will be identical to that described above, for a sandwich assay. In the second, or truly competitive, format the conjugate and sample are mixed together prior to contacting with the bound member of the binding pair. This requires addition of an accurate amount of sample to the conjugate. Such addition can be made directly to the dried conjugate to cause its reconstitution. Thus, in this assay, the format is slightly changed, since wells 38 and 40 are now combined as one well. The other wells remain the same.

In a nucleic acid assay, with a DNA or RNA probe the dipstick is provided with a nitrocellulose membrane either having a bound nucleic acid, or able to bind a sample nucleic acid. The workstation is provided with dried reagents suitable for allowing hybridization of sample nucleic acid to the test nucleic acid and for detecting this hybridization using any one of a variety of techniques. Such techniques include detection of enzyme-labeled nucleic acid, or detecting nucleic acid labeled with radioisotopes, or chemiluminescently labeled, or labeled with reagents which affect the conductivity of a solution e.g., when hydrogen peroxide (substrate) is used with horse radish peroxidase (enzyme), the hydrogen peroxide is broken down and so changes the conductivity of the solution. Since high temperatures are needed in such assays, the workstation must ether be placed on a hot plate, or its equivalent, or an internal heating means provided. One such heating means involves providing two reagents which when mixed react together in an exothermic reaction. These can be caused to be mixed automatically during the assay procedure. The heat from this reaction is used to warm the assay components. Such exothermic reagents include diatomaceous earth or sodium hydroxide mixed with water. The nucleic acid test is especially useful for assay for Johne's disease in farm animals (where the concentration of causative organisms, and thus their nucleic acid, is high) and will allow the assay to be performed at the farm with results within 20 minutes to 2 hours. For example, cells are disrupted in a high salt buffer at 60°C, contacted with the device to allow binding of single stranded nucleic acid from the cells to the membrane, the membrane blocked with excess BSA, contacted with a labeled probe at 37°C in standard hybridization buffer, and binding of this probe detected.

Measuring devices can be adapted to measure the reaction on dipstick 20 according to conductivity, chemiluminescence, or radioactivity. For example, the conductivity of the membrane surface can be measured using a silicon wafer biosensor; and the chemiluminescence or radioactivity of the filter can be used to expose special films, placed within a lightproof box in the workstation. If the film is instant film it can be pulled out of the workstation and immediately developed.

## Claims

1. A kit suitable for assay of a member of a specific binding pair in a sample, said assay entailing performance of a plurality of steps, CHARACTERISED IN THAT the kit comprises a first unit comprising at least one dry reagent, suitable for use in one step of the assay, bound to a solid surface, a second unit comprising a plurality of wells of which one well is adapted to hold liquid suitable for reconstituting the dry reagent, the units being adapted to be capable of being brought together so that the dry reagent and the adapted well are brought in close proximity so that the dry reagent is reconstituted by a said liquid before or during one said step.

2. A kit according to Claim 1 CHARACTERISED IN THAT the units, when brought together, form a final workstation adapted to provide a reagent for each step of the assay, AND BY including a test device adapted to be contacted with each well of the second unit to carry out said steps.

3. A kit according to Claim 1 or Claim 2 CHARACTERISED IN THAT the units are joined together by a hinge which allows the first unit to be positioned over the second unit.

4. A kit according to any preceding Claim CHARACTERISED IN THAT the second unit includes separating means positioned over the adapted well to prevent evaporation of liquid therein.

5. A kit according to any preceding Claim CHARACTERISED IN THAT the adapted well comprises absorbent material for preventing liquid from spilling therefrom.

6. A kit according to any preceding Claim CHARACTERISED IN THAT the adapted well comprises a plastic grid to aid in mixing the dry reagent and a said liquid.

7. A kit according to any preceding Claim CHARACTERISED IN THAT the first unit comprises a well adapted to hold said sample.

8. A kit according to any preceding Claim CHARACTERISED IN THAT the dry reagent is held within a liquid permeable solid material.

9. A kit according to any preceding Claim CHARACTERISED IN THAT the second unit comprises a well adapted to hold a said sample, and wherein the first unit comprises a second well adapted to fit within said sample well, the second well comprising a prefilter positioned to filter liquid from said well to said second well.

10. A kit according to any preceding Claim CHARACTERISED IN THAT the first and second units can be brought together in only one orientation, such orientation ensuring that the dry reagent and a said liquid are brought in close proximity to each other.

11. A kit according to any preceding Claim CHARACTERISED IN THAT said well of the second unit includes felt material comprising said liquid AND BY including a test device which, when brought in contact with the dry reagent in the first unit such contact causes said reconstitution.

12. A kit according to any preceding claim CHARACTERISED IN THAT the first unit comprises at least two dry reagents, and the second unit comprises four wells, including a sample well.

13. A kit according to Claim 12 CHARCTERISED IN THAT the second unit comprises a fifth well comprising a stop solution.

14. A kit according to any preceding Claim CHARACTERISED BY including a dipstick test device for detecting an analyte in a liquid sample by treating the analyte with at least one liquid reagent to form a detectable reaction product, which dipstick test device comprises:

a) means defining an aqueous permeable, aqueous insoluble reaction zone, adapted to retain said detectable reaction product, the means defining a reaction zone comprising a first region exposed to receive said liquid sample and a second region, spaced from the first region;

b) a control absorbent in liquid-transferring relation with the second region of the defining means, which control absorbent has a predetermined, limited, standard, liquid-absorbing capacity, the control absorbent and the defining means being positioned in the device in the manner that the control absorbent, in being filled to capacity, effectively meters a predetermined limited flow between the first region and the second region, said predetermined, limited, standard volume being selected to enable reliable detection of said analyte.

15. A kit according to Claim 14 CHARACTERISED BY including

c) an absorbent reservoir;

d) means retaining the absorbent reservoir in a first position spaced apart from liquid-transferring contact with the control absorbent; and

e) means for moving the absorbent reservoir from said first position to a second position, in liquid-transferring contact with the control absorbent; whereby the control absorbent controls the sample volume absorbed into the defining means while the absorbent reservoir is in said first position; thereafter the absorbent reservoir is moved into said second position, and the defining means is contacted with at least one liquid reagent which is drawn into said absorbent reservoir.

16. A method for performing an assay for a member of a specific binding pair in a sample, said assay entailing performance of a plurality of steps, CHARACTERISED BY the steps of:
providing a first unit comprising at least one dry reagent, suitable for use in one step of the assay bound to a solid surface,
providing a second unit comprising a plurality of wells of which one well is adapted to hold liquid for reconstituting the dry reagent, or is of use in one said step.
positioning the first unit and second units together so that the dry reagent and the adapted well are brought in close proximity, providing a test device which may be positioned over or within each well of the second unit, and positioning the test device over said well comprising said liquid to allow reaction of the test device with the dry reagent and said liquid after the dry reagent and said liquid have been reconstituted.

17. A method according to Claim 16 CHARACTERISED BY including the step of positioning the test device in each said well in a predetermined order to carry out each step of the assay.

07·04·89

FIG.1

FIG.2

07·04·89

FIG.3

FIG.4

FIG.5

FIG.6

←—61

←—220

←—61

FIG.7

7A  7A                          14  7B    7B
54 58    44        36    16         66
40                42        54   46      64   64   64
      56                            24 64  26   28   30
          54
      38            48              22    20

56  58

40
62
60

FIG.7A

66

24

64

FIG.7B

210

208

206

203

FIG.10

204

202          207
205      200

07·04·89

FIG.8

FIG.9